Europäisches Patentamt

European Patent Office

Office européen des brevets

(11) Veröffentlichungsnummer: **0 242 491**
**A1**

## (12) EUROPÄISCHE PATENTANMELDUNG

(21) Anmeldenummer: **86810223.7**

(22) Anmeldetag: **23.05.86**

(51) Int. Cl.⁴: **A61M 1/00**

(30) Priorität: **24.04.86 CH 1684/86**

(43) Veröffentlichungstag der Anmeldung:
**28.10.87 Patentblatt 87/44**

(84) Benannte Vertragsstaaten:
**AT DE FR GB**

(71) Anmelder: **Genossenschaft VEBO**
**Solothurnische Eingliederungsstätte für**
**Behinderte**
**Werkhofstrasse**
**CH-4702 Oensingen(CH)**

(72) Erfinder: **Hauri, Hermann**
**Fliederweg 6**
**CH-5600 Lenzburg(CH)**

(74) Vertreter: **Eder, Carl E. et al**
**Patentanwaltsbüro EDER AG**
**Münchensteinerstrasse 2**
**CH-4052 Basel(CH)**

(54) **Saugvorrichtung für die Wunddrainage und Verwendung der Saugvorrichtung.**

(57) Die Saugvorrichtung weist ein Sauggefäss (1) mit einer Decke (5) auf, die mit einem Ansatzteil (5b) versehen ist. An dessen Deckteil (5d) ist eine gummielastische Membran (11) befestigt. Die Decke (5) besitzt zwei Anschlüsse (5a, 5f), deren Durchgangsöffnungen mit dem Sauggefäss-Innenraum (7) bzw. der vom Ansatzteil (5b) und der Membran (11) begrenzten Kammer (15) verbunden sind. Die beiden Anschlüsse (5a, 5f) werden spätestens kurz nach der Evakuierung des Sauggefäss-Innenraums (7) durch einen Verbindungsschlauch (19) miteinander verbunden, so dass in der Kammer (15) der gleiche Unterdruck vorhanden ist wie im Sauggefäss-Innenraum (7), bis der Verbindungsschlauch (19) für die Durchführung einer Wunddrainage vom zur Kammer gehörenden Anschluss (5f) getrennt und mit einer zur Wunde führenden Saugleitung verbunden wird. Dadurch kann auch bei einer dünnen Membran (11) vermieden werden, dass diese während der zwischen der Bereitstellung des evakuierten Sauggefässes (1) und dessen Benutzung für eine Wunddrainge verstreichenden Aufbewahrungszeit ermüdet und dass während der letzeren Gas durch die Membran (11) in den Sauggefäss-Innenraum (7) hinein diffundiert.

FIG. 1

## Saugvorrichtung für die Wunddrainage und Verwendung der Saugvorrichtung

Die Erfindung betrifft eine Saugvorrichtung gemäss dem Oberbegriff des Anspruchs 1.

Eine aus der Schweizerpatentschrift Nr. 605 313 bekannte Saugvorrichtung weist ein Sauggefäss auf, das oben eine mit einem Stopfen verschlossene Öffnung besitzt. Der aus einem gummiartigen Material bestehende Stopfen hat einen mit ihm zusammenhängenden, mit einer Klemme verschliessbaren Verbindungsschlauch zum Anschliessen einer Saugleitung und einen zu einer Membran verdünnten Abschnitt mit zwei nach aussen ragenden Hörnern. Die Membran bildet zusammen mit den Hörnern eine Anzeigevorrichtung zum Anzeigen der zwischen der Umgebung und dem Sauggefäss-Innenraum vorhandenen Druckdifferenz, d.h. des im Innenraum vorhandenen Unterdrucks. Wenn nämlich im Innenraum des Sauggefässes gegenüber der Umgebung ein Unterdruck herrscht, wird die im entspannten Zustand ebene Membran nach innen gebogen, so dass die Stellung der mit der Membran verbundenen Hörner ein Mass für den Wert des Unterdrucks gibt. Die Membran wird während ihrer Aufbewahrung vom Zeitpunkt der Evakuation des Sauggefässes bis zu dessen Benutzung für eine Wunddrainage dauernd deformiert, wodurch die Membran ermüdet und/sogar plastisch deformiert werden kann. Wenn nun das Sauggefäss für eine Wunddrainage benutzt wird, kehrt die Membran nicht mehr genau bis in die ursprüngliche, entspannte Lage zurück, wodurch der Unterdruck falsch angezeigt wird. Dieser Fehler bei der Anzeige des Unterdrucks wird umso grösser, je grösser die Zeitdauer von der Evakuierung des Sauggefässes bis zu dessen Benutzung für die Wunddrainage ist. Anzeigevorrichtungen, die gemäss der Schweizerpatentschrift Nr. 605 313 oder ähnlich ausgebildet sind, wären insbesondere auch nicht für Sauggefässe geeignet, die nur für einen einmaligen Gebrauch vorgesehen sind, bei denen die erwähnte Zeitdauer häufig mehrere Wochen oder Monate beträgt. Die Genauigkeit bei der Ermittlung des Wertes des Unterdrucks wird bei der Saugvorrichtung gemäss der Schweizerpatentschrift Nr. 605 313 zudem noch dadurch reduziert, dass die durch den Unterdruck bewirkte Deformation der Membran wegen des verhältnismässig grossen Verhältnisses zwischen Dicke und Oberfläche der Membran relativ gering ist. Wenn man das Verhältnis zwischen Dicke und Oberfläche kleiner machen würde, ergäbe sich während der Aufbewahrungszeit des evakuierten Sauggefässes eine noch stärkere Ermüdung und plastische Deformation der Membran. Zudem steigt mit abnehmender Membrandicke die Gefahr, dass Gas durch die Membran hindurch in den Sauggefäss-Innenraum hineindiffundiert. Ferner besitzt die Anzeigevorrichtung der bekannten Saugvorrichtung keinerlei Skala, was eine einigermassen genaue Druckermittlung noch zusätzlich erschwert.

Der Erfindung liegt daher die Aufgabe zugrunde, eine Saugvorrichtung zu schaffen, die Nachteile der bekannten Saugvorrichtung behebt und vor allem ermöglicht, die zwischen der Umgebung und dem Innenraum des Sauggefässes vorhandene Druckdifferenz genauer zu ermitteln, wobei die Anzeigevorrichtung und die ganze Saugvorrichtung kostengünstig herstellbar sein soll, so dass die letztere insbesondere auch für den einmaligen Gebrauch vorgesehen werden kann.

Diese Aufgabe wird durch eine Saugvorrichtung der einleitend genannten Art gelöst, wobei die Saugvorrichtung erfindungsgemäss durch den kennzeichnenden Teil des Anspruchs 1 gekennzeichnet ist. Vorteilhafte Ausgestaltungen der Saugvorrichtung gehen aus den vom Anspruch 1 abhängigen Ansprüchen hervor.

Die Erfindung betrifft ferner eine Verwendung der Saugvorrichtung gemäss dem Oberbegriff des Anspruchs 7, wobei die Verwendung erfindungsgemäss durch den kennzeichnenden Teil des Anspruchs gekennzeichnet ist.

Die erfindungsgemässe Saugvorrichtung kann zwischen der mindestens teilweisen Evakuierung ihres Sauggefäss-Innenraums und ihrer Benutzung zum Absaugen von Wundsekret in einem Zustand aufbewahrt werden, in dem über der Membran, d.h. zwischen der Kammer und dem Sauggefäss-Innenraum keine Druckdifferenz vorhanden ist. Dadurch kann auch bei verhältnismässig geringer Membrandicke sichergestellt werden, dass die Membran während der Aufbewahrung des evakuierten Sauggefässes ihre Elastizität beibehält und nicht ermüdet und dass während der genannten Aufbewahrung weder Luft noch eine von deren Komponenten durch die Membran hindurch in den Sauggefäss-Innenraum hinein diffundiert. Die Saugvorrichtung kann daher ohne Nachteile einige Wochen oder Monate oder sogar bis ein Jahr mit evakuierten Sauggefäss aufbewahrt werden. Die Möglichkeit, die Membran im Vergleich zur Oberfläche ihres durch die zwischen der Kammer und dem Sauggefäss-Innenraum vorhandene Druckdifferenz deformierbaren Teils und auch in absoluten Masseinheiten verhältnismässig dünn auszubilden, ergibt wiederum den Vorteil, dass die Membran durch zwischen der Kammer und dem Sauggefäss-Innenraum vorhandene Druckdifferenzen verhältnismässig stark deformiert wird, so dass die Grössen oder Werte der Druckdifferenz gut ablesbar sind.

Der Erfindungsgegenstand wird nun anhand eines in der Zeichnung dargestellten Ausführungsbeispiels erläutert. In der Zeichnung zeigt

die Figur 1 einen Vertikalschnitt durch das Sauggefäss einer Saugvorrichtung im Zustand, in dem auf beiden Seiten der Membran der gleiche Druck vorhanden ist, und

die Figur 2 einen Vertikalschnitt durch das Sauggefäss der in der Figur 1 dargestellten Saugvorrichtung, aber mit durch eine Druckdifferenz deformierter Membran.

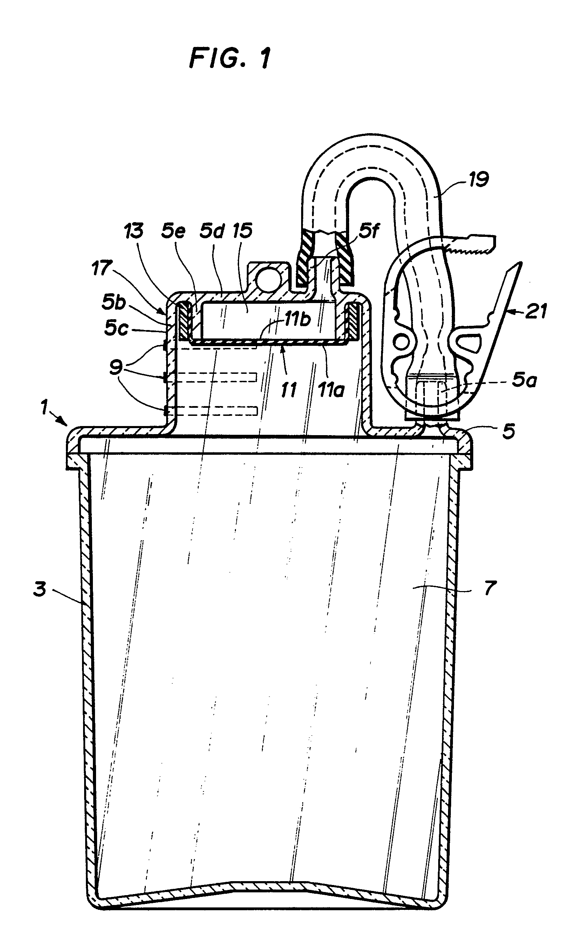

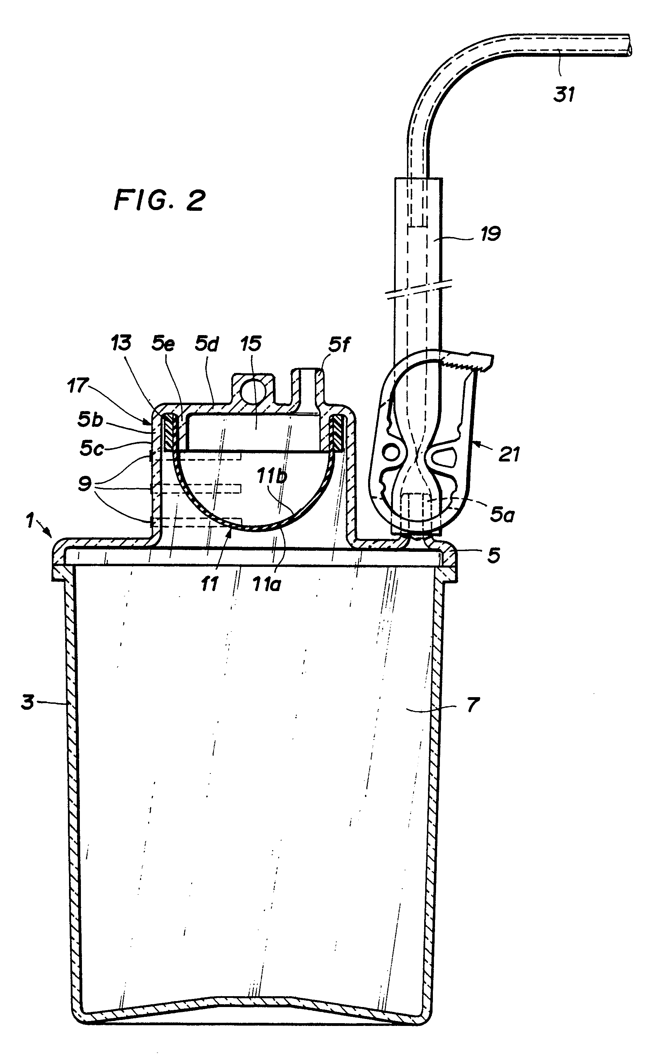

Die in den Figuren 1 und 2 dargestellte Saugvorrichtung für die Wunddrainage weist ein für den einmaligen Gebrauch vorgesehenes Sauggefäss 1 auf. Dieses, oder genauer gesagt, dessen Wandung ist aus zwei Teilen, nämlich einem Topf 3 und einer starr sowie unlösbar mit diesem verbundenen, nämlich verschweissten Decke 5 gebildet und begrenzt einen Innenraum 7. Die Decke 5 besitzt in der Nähe ihres Randes einen durch einen Stutzen gebildeten Anschluss 5a und in der Mitte einen stutzenförmigen Ansatzteil 5b, der vom an ihn anschliessenden Teil der Decke 5 weg auf die Aussenseite des Sauggefässes 1, d.h. nach oben ragt. Der Ansatzteil 5b weist einen kreiszylindrischen Mantelteil 5c und einen ebenen Deckteil 5d auf. Der letztere ist mit einem ringförmigen, in den Innenraum 7 hineinragenden Teil 5e und einem Anschluss 5f, nämlich einem vom Innenraum 7 weg nach oben ragenden Stutzen versehen. Der Topf 3 und die Decke 5 bestehen beide aus einem farblosen, durchsichtigen, glasklaren und auch bei der Sterilisation mit Gammastrahlen durchsichtig sowie glasklar bleibenden, formfesten Kunststoff, etwa den unter dem Handelsnamen ARNITE erhältlichen Copolyester. Der Ansatzteil 5b oder, genauer gesagt, dessen Mantelteil 5c dient noch als Skalenträger und ist mit einer Skala 9 versehen, die beispielsweise drei entlang der Mittelachse des Mantelteils 5c voneinander beabstandete, die genannte Mittelachse zumindest zum Teil bogen oder ringförmig umschliessende, farbige, beispielsweise aufgedruckte Markierungen aufweisen kann.

Eine zur Mittelachse des Ansatzteils 5b rotationssymmetrische, elastische, aus einem farbigen, zum Beispiel orangen, gummielastischen Material, nämlich Nitrilkautschuk bestehende Membran 11 liegt mit einem Randabschnitt an der äusseren, zylindrischen Fläche des ringförmigen Teils 5e an und ist an diesem mit einem aufgepressten Ring 13 festgeklemmt. Der dem Deckteil 5c abgewandte Rand des Rings 13 ist mindestens ungefähr bündig mit dem freien Rand des ringförmigen Teils 5e. Der Ring 13 besteht aus einem mindestens einigermassen formfesten Kunststoff, zum Beispiel aus dem gleichen Copolyester wie das Sauggefäss 1, ist jedoch vorzugsweise zumindest einigermassen undurchsichtig und hat zum Beispiel eine weissliche Farbe, so dass er den befestigten Randabschnitt der Membran 11 abdeckt. Die eine, erste Seite oder Fläche 11a des mittleren, freien, d.h. nicht zwischen dem ringförmigen Teil 5e und dem Ring 13 festgeklemmten Abschnitts der Membran 11 grenzt an den Innenraum 7 des Sauggefässes 1 an. Die andere, zweite, dem Innenraum 7 abgewandte Seite oder Fläche 11b des genannten, mittleren Abschnitts der Membran 11 begrenzt zusammen mit dem Ansatzteil 5b der Sauggefässwandung oder, genauer gesagt, dem Deckteil 5d und dem ringförmigen Teil 5e eine Kammer 15. Diese befindet sich zwar auch im Innern der Sauggefäss-Wandung, hat jedoch ein wesentlich kleineres Volumen als der eigentliche, von ihr durch die Membran 11 getrennte Innenraum 7 des Sauggefässes 1. Der Anschluss 5a, oder genauer gesagt, dessen Durchgangsöffnung ist fluidmässig mit dem Sauggefäss-Innenraum verbunden, während der Anschluss 5f oder, genauer gesagt, dessen Durchgangsöffnung fluidmässig mit der Kammer 15 verbunden ist. Im übrigen haben die im wesentlichen zylindrischen, die Anschlüsse 5a und 5f bildenden Stutzen beide zumindest annähernd die gleichen Aussendurchmesser. Wie noch näher erläutert wird, bildet der stutzenförmige Teil 5b des Sauggefässes zusammen mit der Membran 11 eine Anzeigevorrichtung 17 zum Anzeigen der zwischen der Kammer 15 und dem Sauggefäss-Innenraum 7 vorhandenen Druckdifferenz, d.h. des Unterdrucks im Innenraum 7.

Am fluidmässig mit dem Sauggefäss-Innenraum 7 verbunde nen Anschluss 5a ist das eine Ende eines Verbindungsschlauchs 19 dicht und lösbar befestigt, nämlich angesteckt. Der Verbindungsschlauch 19 besteht aus einem weichen Polyvinylchlorid oder Polyurethan. Eine aus einem elastisch deformierbaren, thermoplastischen Kunststoff bestehende, am Verbindungsschlauch 19 gehaltene Klemme 21 hat zwei vom Verbindungsschlauch 19 durchdrungene Löcher, an einem Endabschnitt eine Anzahl Riller und am anderen Ende einen schneidenartigen Rand, der lösbar in eine der Rillen eingerastet werden kann. Wenn die beiden Enden der Klemme 21 wie in der Figur 1 dargestellt, voneinander getrennt sind, gibt die Klemme den Durchgang des Verbindungsschlauch 19 frei. Wenn hingegen das schneidenartig ausgebildete Ende der Klemme 19 gemäss der Figur 2 in eine Rille des anderen Klemme-Endabschnitts eingerastset ist, klemmt und quetscht sie den Verbindungsschlauch 19 derart zusammen, dass dessen Durchgang dicht gesperrt wird.

Das dem Anschluss 5a abgewandte Ende des Verbindungsschlauchs 19 kann gemäss der Figur 1 lösbar mit dem Anschluss 5f oder mit der in der Figur 2 dargestellten Saugleitung 21 verbunden werden. Die Saugleitung 31 weist mindestens einen Schlauch und im allgemeinen zwei durch ein Kupplungsstück miteinander verbundene Schläuche auf, wobei der zum Einführen in den Körper eines Patienten bestimmte Schlauchabschnitt mit Drainagelöchern versehen ist.

Nun soll die Herstellung, Bereitstellung und Benutzung der Saugvorrichtung erläutert werden. Nach der Herstellung der verschiedenen Einzelteile wird die Membran 11 mit dem Ring 13 an der Decke 5 befestigt. Danach werden der Topf 3 und die Decke 5 durch Reibschweissen miteinander verbunden, der Verbindungsschlauch 17 am Anschluss 5a befestigt und die Klemme 17 am Verbindungsschlauch 15 angebracht. Das dem Anschluss 5a abgewandte Ende des Verbindungsschlauchs 19 kann nun mit einer nicht dargestellten Pump-und Evakuiereinrichtung verbunden werden, wonach der Innenraum 7 durch den Durchgang des Verbindungsschlauchs 19 hindurch mehr oder weniger stark evakuiert werden kann. Die Klemme 21 befindet sich während der Evakuation selbstverständlich in derjenigen Stellung, in der sie den Durchgang des Verbindungsschlauchs 19 frei gibt, und wird nach der Evakuation zugeklemmt, so dass sie den genannten Durchgang sperrt. Das dem Anschluss 5a abgewandte Ende des Verbindungsschlauchs 19 kann nun von der Evakuiereinrichtung getrennt und sofort oder relativ bald, beispielsweise spätestens nach einigen Stunden oder Tagen, an den Anschluss 5f angesteckt werden. Daraufhin wird die Klemme 19 in die in der Figur 1 dargestellte Stellung gebracht, in der sie den Durchgang des Verbindungsschlauchs 19 wieder freigibt. Es strömt nun Luft aus der Kammer 15 und dem Verbindungsschlauch-Durchgang in den Sauggefäss-Innenraum 7, so dass zwischen diesem und der Kammer 15 ein Druckausgleich stattfindet, bei welchem der Druck im Sauggefäss-Innenraum 7 wieder ein wenig ansteigt, aber immer noch wesentlich kleiner bleibt als der in der Umgebung des Sauggefässes herrschende Luftdruck. Nach dem genannten Druckausgleich ist zwischen der Umgebung des Sauggefässes 1 und dessen Innenraum ungefähr eine vorgesehene Nenn-oder Anfangs-Druckdifferenz vorhanden. Da die zwischen der Kammer 15 und dem Innenraum 7 vorhandene Druckdifferenz beim genannten Druckausgleich vollständig verschwindet, d.h. Null wird, nimmt die Membran 11 nach dem Druckausgleich die in der Figur 1 dargestellte Form an, in der der mittlere, freie Abschnitt der Membran in der vom freien Rand des ringförmigen Abschnitts 5e definierten Ebene liegt, einen Kreis bildet und entspannt ist oder höchstens unter einer relativ geringen, durch das Festklemmen des Membran-Randabschnitts erzeugten und definierten Spannung steht. Die Saugvorrichtung wird nun in diesem Zustand in eine Kunststoffhülle eingepackt und die letztere luftdicht zugeschweisst. Danach wird die Saugvor richtung durch Bestrahlen mit Gammastrahlen steril gemacht.

Die Saugvorrichtung kann nun zum Beispiel einem Spital zugestellt und in diesem einige Tage, Wochen oder Monate aufbewahrt werden, bis sie für die Drainage einer Wunde, insbesondere bei oder nach einer Operation für die Drainage einer Operationswunde verwendet werden soll. Wenn eine solche Wunddrainage durchgeführt werden soll, wird die erwähnte Hülle von der Saugvorrichtung entfernt, der Verbindungsschlauch-Durchgang durch Zuklemmen der Klemme 21 gesperrt und das am Anschluss 5f steckende Ende des Verbindungsschlauchs 19 vom Anschluss 5f getrennt. Sobald der Verbindungsschlauch 19 vom Anschluss 5f getrennt ist, strömt durch dessen Durchgangsöffnung Luft aus der Umgebung in die Kammer 15 ein. Dadurch wird der Druck in der letzeren grösser als im Innenraum 7, so dass die Membran 11 oder, genauer gesagt, deren mittlerer, freier Abschnitt gedehnt sowie deformiert und in der in der Figur 2 gezeichneten Weise zum Sauggefäss-Innenraum hin gewölbt wird. Ferner kann nun die Saugleitung 31 mit ihrem einen Ende in die Operationswunde und mit ihrem anderen Ende in den Verbindungsschlauch 19 gesteckt werden. Das Sauggefäss kann des weitern mit seinem in der Mitte des Deckteils 5d vorhandenen, ösenartigen Ansatz an einem Haken oder dergleichen aufgehängt werden.

Wenn infolge des im Sauggefäss-Innenraum 7 herrschenden Unterdrucks nun flüssiges Wundsekret und eventuell auch noch in diesem enthaltene Feststoffteilchen und/oder Luft und/oder anderes Gas in den Sauggefäss-Innenraum 7 eingesaugt wird, steigt der im letzeren vorhandene Druck gegen den Umgebungs-Luftdruck an. Dadurch nehmen die Dehnung und Deformation der Membran 11 ab, so dass deren Form sich wieder langsam der in der Figur 1 dargestellten Form nähert. Die Membran 11 der Anzeigevorrichtung 17 zeigt in Zusammenwirkung mit der Skala 9 die zwischen der Kammer 15 und dem Sauggefäss-Innen raum 7 vorhandene Druckdifferenz an. Eine die Saugvorrichtung kontrollierende Person kann die Membran 11 durch den Mantelteil 5c hindurch sehen und so den Wert der genannten Druckdifferenz ablesen. Wenn die Druckdifferenz auf Null oder einen anderen vorgesehenen Minimalwert abgesunken ist, zum Beispiel wenn der mittlere, durch die Druckdifferenz deformierbare Membranabschnitt zur sich in den Figuren 1 und 2 zuoberst befindenden Skalen-Markierung oder in deren Nähe gelangt, kann eine den Patienten betreuende Person den Verbindungsschlauch 19 mit der Klemme 21 zuklemmen sowie von der Saugleitung 31 trennen und nötigenfalls ein anderes, evakuiertes Sauggefäss mit der Saugleitung 31 verbinden.

Der Durchmesser der Aussenfläche des ringförmigen, zylindrischen Teils 5e und damit auch der Durchmesser, den der mittlere, durch die Druckdifferenz deformierbare Abschnitt der Membran 11 in der Figur 1 dargestellten Zustand hat, beträgt mindestens 20 mm und beispielsweise 25 bis 35 mm. Die Membran 11 kann ohne weiteres verhältnismässig dünn ausgebildet werden und eine höchstens 2 mm, vorzugsweise höchstens 1 mm und zum Beispiel 0,3 bis 0,9 mm betragende Dicke haben. Die Dicke der Membran 11 ist also höchstens 10% und vorzugsweise höchstens 5% des Durchmesser des Kreises, den der mittlere, deformierbare Abschnitt der Membran im in der Figur 1 dargestellten Zustand einnimmt. Da der letztgenannte Durchmesser im Vergleich zur Dicke der Membran und auch in absoluten Masseinheiten relativ gross ist, wird die Membran 11 im Fall, dass die zu ermitelnde Druckdifferenz ihren Nenn-oder Anfangs-Wert hat, verhältnismässig stark vom freien Rand des ringförmigen Teils 5e zum Innenraum 7 hin gewölbt. Der Weg, den das Zentrum der Membran 11 zwischen deren beiden in den Figuren 1 und 2 gezeichneten Extrem-Formen zurücklegt, beträgt mindestens 7 mm, vorzugsweise mindestens 10 mm und beispielsweise ungefähr 15 mm. Zudem ist die Membran rund um den Mantelteil 5c herum durch diesen hindurch ersichtlich. Die An zeigevorrichtung 17 erlaubt also einer Person, den Wert der zwischen der Kammer 15 und dem Innenraum 7 vorhandenen Druckdifferenz mühelos und verhältnismässig genau abzulesen.

Die an den beiden Enden der Skala 9 vorhandenen Markierungen können zum Beispiel mit "MAX" und "MIN" bezeichnet werden. Im übrigen kann die weiter vorne erwähnte Nenn-oder Anfangs-Druckdifferenz zwischen der Umgebung und dem Sauggefäss-Innenraum 7 abhängig von der Empfindlichkeit des die zu behandelnde Wunde enthaltenden Körperbereichs verschieden festgelegt. werden. Man kann zum Beispiel zwei Typen von Saugvorrichtungen bereitstellen, wobei die Nenn-oder Anfangs-Druckdifferenz beim ersten Typ ungefähr 90 und beim zweiten Typ ungefähr 30 Kilopascal betragen kann. Die Skalen 9 können in diesem Fall für die beiden Typen mit verschiedenen Markierungs-Abständen hergestellt werden, so dass sich das Zentrum der Membran beim Vorhandensein der Nenn-oder Anfangs-Druckdifferenz trotz verschiedenen Membran-Deformationen bei beiden Typen bei der am weitesten vom Deckteil 5d entfernten Markierung befindet. Stattdessen könnte man die beiden Typen auch mit verschieden dicken Membranen ausrüsten, so dass die Membran-Zentren beim Vorhandensein der Nenn-oder Anfangs-Druckdifferenz bei beiden Typen ungefähr gleich stark ausgelenkt werden.

Das Verfahren für die Herstellung und Evakuierung der Sauggefässe könnte dahingehend geändert werden, dass man die beiden Anschlüsse 5a und 5f bereits fluidmässig mit dem Verbindungsschlauch 19 verbindet, bevor der Topf 3 und die Decke 5 miteinander verschweisst sind. Die beiden Sauggefäss-Teile könnten in diesem Fall in einem auf den vorgesehenen Nenn-oder Anfangs-Unterdruck evakuierten Holraum einer Produktionseinrichtung miteinander verschweisst werden, so dass schon unmittelbar bei der Herstellung des evakuierten Sauggefässes in der Kammer 15 der gleiche Unterdruck vorhanden ist wie im Innenraum 7.

Die Saugvorrichtungen selbst können ebenfalls in verschiedener Hinsicht geändert werden. Zum Beispiel könnte die Membran noch mit mindestens einem mittig oder aussermittig angeordneten und mit ihr zusammenhängenden oder sonst verbundenen horn-und/oder stäbchen-und/oder rutenartigen Anzeige-Element versehen werden, das beim Deformieren der Membran verschoben und bei aussermittiger Anordnung vor allem auch noch verschwenkt wird. Das Anzeige-Element kann entweder auf der dem Sauggefäss-Innenraum oder der der Kammer zugewandten Membranseite angeordnet werden, sich vollständig innerhalb der Sauggefäss-Wandung befinden und durch einen durchsichtigen Bereich von dieser hindurch ersichtlich sein. Bei sich auf der an die Kammer angrenzenden Membranseite befindendem Anzeige-Element könnte man dieses und den zur Kammer gehörenden Anschluss-Stutzen koaxial zur durch das Membranzentrum verlaufenden Mittelachse des die Membran enthaltenden Ansatzteils des Sauggefässes anordnen und die Länge des Anzeige-Elements derart bemessen, dass es die Kammer sowie die Durchgangsöffnung des Stutzens durchdringt und aus diesem heraus in die Umgebung des Sauggefässes ragt. Wenn der Verbindungsschlauch vom zur Kammer gehörenden Anschluss-Stutzen getrennt ist, wäre der aus dem letzteren herausragende Teil des Anzeige-Elements dann für eine Person direkt, d.h. ohne durch die Sauggefäss-Wandung hindurch zu sehen, sichtbar. Wenn ein in der einen oder anderen Weise angeordnetes und ausgebildetes Anzeige-Element vorhanden ist, könnte dieses gewissermassen als Zeiger dienen, wobei eine entsprechend angepasste Skala vorgesehen werden kann.

Die Saugvorrichtung kann auch für einen mehrmaligen Gebrauch des Sauggefässes konzipiert werden. Das letztere kann in diesem Fall mit einer Öffnung versehen werden, die mit einem entfernbaren, zum Beispiel stopfen-oder deckelartigen Verschlusselement verschliessbar ist. Die Anzeigevorrichtung kann dann entweder direkt an der Wandung des eigentlichen Sauggefässes oder am Verschlusselement angeordnet werden.

**Ansprüche**

1. Saugvorrichtung für die Wunddrainage, mit einem Sauggefäss (1), einem an diesem befestigten Verbindungsschlauch (19) zum Verbinden des Sauggefäss-Innenraums (7) mit einer Saugleitung (31), einer Klemme (21) zum Zuklemmen des Verbindungsschlauchs (19) und einer zum Anzeigen der Druckdifferenz zwischen der Umgebung und dem Innenraum (7) dienende Anzeigevorrichtung (17) mit einer am Sauggefäss (1) gehaltenen, elastisch deformierbaren Membran (11), die eine erste, an den genannten Innenraum (7) angrenzende Fläche (11a) und eine zweite, diesem abgewandte Fläche (11b) besitzt, dadurch gekennzeichnet, dass die zweite Fläche (11b) der Membran (11) zusammen mit einem Teil (5b) des Sauggefässes (1) eine Kammer (15) begrenzt und dass der genannte Sauggefäss-Teil (5b) einen Anschluss (5f) aufweist, mit dem das zum Verbinden mit der Saugleitung (31) bestimmte Ende des Verbindungsschlauchs (19) lösbar verbindbar ist.

2. Saugvorrichtung nach Anspruch 1, dadurch gekennzeichnet, dass die Membran (11) und/oder ein mit dieser verbundenes Element durch einen durchsichtigen Bereich des Sauggefässes (1) hindurch von dessen Umgebung her sichtbar ist.

3. Saugvorrichtung nach Anspruch 1 oder 2, dadurch gekennzeichnet, dass der Anschluss (5f) des genannten Sauggefäss-Teils (5b) als Stutzen ausgebildet ist, an oder eventuell in den der Verbindungsschlauch (19) lösbar steckbar ist.

4. Saugvorrichtung nach einem der Ansprüche 1 bis 3, dadurch gekennzeichnet, dass der genannte Sauggefäss-Teil (5b) einen zylindrischen, durchsichtigen, die Membran (11) umschliessenden Mantelteil (5c) besitzt und dass die Membran (11) zur Mittelachse dieses Mantelteils (5c) rotationssymmetrisch ist.

5. Saugvorrichtung nach einem der Ansprüche 1 bis 4, dadurch gekennzeichnet, dass der genannte Sauggefäss-Teil (5b) als Skalenträger dient.

6. Saugvorrichtung nach einem der Ansprüche 1 bis 5, dadurch gekennzeichnet, dass der Rand der Membran (11) mit einem Ring (13) an einer ringförmigen Fläche des genannten Sauggefäss-Teils (5b) festgeklemmt ist.

7. Saugvorrichtung nach einem der Ansprüche 1 bis 6, dadurch gekennzeichnet, dass der genannte Sauggefäss-Teil (5b) mit der restlichen Wandung des Sauggefässes (1) unlösbar verbunden ist.

8. Verwendung der Saugvorrichtung nach einem der Ansprüche 1 bis 7, wobei der Innenraum (7) des Sauggefässes (1) vor dessen Benutzung für eine Wunddrainage zumindest teilweise evakuiert wird, dadurch gekennzeichnet, dass der Sauggefäss-Innenraum (7) spätestens nach dem Evakuieren durch den Verbindungsschlauch (19) mit der Kammer (15) verbunden wird und dass der Verbindungsschlauch (19) vor der Benutzung des Sauggefässes (1) für eine Wunddrainage mit der Klemme (21) zugeklemmt und sein mit dem genannten Anschluss (5f) verbundenes Ende danach vom Anschluss (5f) getrennt wird.

## FIG. 1

FIG. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| . A | EP-A-0 066 699 (INTERMEDICAT GmbH)<br>* Figuren; Seite 7, Zeilen 10-13 *<br><br>----- | 1 | A 61 M 1/00 |
| | | | **RECHERCHIERTE SACHGEBIETE (Int. Cl.4)**<br><br>A 61 M |

Der vorliegende Recherchenbericht wurde für alle Patentansprüche erstellt.

| Recherchenort | Abschlußdatum der Recherche | Prüfer |
|---|---|---|
| DEN HAAG | 11-02-1987 | VEREECKE A. |

KATEGORIE DER GENANNTEN DOKUMENTE
X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder
    nach dem Anmeldedatum veröffentlicht worden ist
D : in der Anmeldung angeführtes Dokument
L : aus andern Gründen angeführtes Dokument

& : Mitglied der gleichen Patentfamilie, überein-
    stimmendes Dokument

EPA Form 1503 03 82